# EUROPÄISCHE PATENTSCHRIFT

(11) **EP 0 666 067 B1**
(45) Veröffentlichungstag und Bekanntmachung des Hinweises auf die Patenterteilung: **08.08.2001**
(21) Anmeldenummer: 94810064.9
(22) Anmeldetag: 07.02.1994
(51) Int. Cl.: A61F 2/34, A61F 2/30

(54) **Mindestens zweischalige Gelenkpfanne einer Hüftgelenkprothese und Hüftgelenkprothese mit einer derartigen Gelenkpfanne**
Acetabular cup of a hip joint prothesis having at least two shells and hip joint prosthesis with such an acetabular cup
Coque acétabulaire d'une prothèse de l'articulation de la hanche à au moins deux cupules et prothèse de l'articulation de la hanche avec une telle coque acétabulaire

(43) Veröffentlichungstag der Anmeldung: 09.08.1995
(73) Patentinhaber: Sulzer Orthopädie AG, 6340 Baar (CH)
(72) Erfinder: Oehy, Jürg, CH-8405 Winterthur (CH); Bider, Kurt, CH-8406 Winterthur (CH); Schoch, Martin, CH-8143 Stallikon (CH)
(74) Vertreter: Sulzer Management AG

(56) Entgegenhaltungen:
- EP-A- 0 313 762
- EP-A- 0 444 381
- EP-A- 0 472 315
- EP-A- 0 490 616
- FR-A- 2 668 055
- US-A- 4 955 325
- US-A- 5 021 062

## Beschreibung

Die Erfindung betrifft eine mindestens zweischalige Gelenkpfanne einer Hüftgelenkprothese entsprechend dem Oberbegriff des Patentanspruchs 1, der der offenbanung der Druckschrift EP-A-0 444 381 entspricht. Ferner betrifft die Erfindung eine mit einer derartigen Gelenkpfanne ausgeführte Hüftgelenkprothese sowie einen Gewindestopfen für eine derartige Gelenkpfanne.

Eine Gelenkpfanne der genannten Art ist aus der US-Patentschrift 5 021 062 bekannt. Eine im Polbereich der Aussenschale der bekannten Gelenkpfanne vorgesehene Gewindebohrung soll einerseits die Positionierung der Aussenschale im Knochengewebe erleichtern und dem Chirurgen, z.B. mittels eines einschraubbaren Halters, eine provisorische Fixierung der Aussenschale im Knochengewebe ermöglichen und andererseits als Führungsbohrung für einen Zentrierteil dienen, welcher von der aus einem Kunststoff bestehenden Innenschale absteht. Bei derartigen Gelenkpfannen können unter Belastung, etwa aufgrund von elastischen Verformungen der zusammenwirkenden Teile, Abriebpartikel entstehen, welche bei der bekannten Ausführung zwischen der Wand der Gewindebohrung und dem in diesem beweglich geführten Zentrierteil in den an die Gewindebohrung anschliessenden Teil des Knochenbetts austreten können.

Der Erfindung liegt die Aufgabe zugrunde, eine insbesondere in dieser Hinsicht verbesserte Gelenkpfanne zu schaffen, welche einen Austritt von Abriebpartikeln in das die Aussenschale umgebende Knochengewebe verhindert.

Diese Aufgabe wird gemäss der Erfindung durch die im Patentanspruch 1 angegebenen Merkmale gelöst.

Durch die erfindungsgemässe Ausführung der Gelenkpfanne wird auf einfache Weise eine sichere Abdichtung des Innenraumes der Aussenschale gegen das Knochenbett gewährleistet. Entsprechend wird ein Kontakt zwischen allfällig entstehenden Abriebpartikeln und dem Knochengewebe verhindert und damit die Gefahr einer Weiterverbreitung derartiger Abriebpartikel, z.B. ein Übertritt in den Blutkreislauf, vermieden. Die erfindungsgemässe Ausführung gestattet zugleich die Bildung einer von äusseren Einflüssen freien Beobachtungsstelle, welche eine Kontrolle des Zustandes des unter der Aussenschale liegenden Teils des Knochenbetts ermöglicht, ohne die Aussenschale aus dem Knochenbett zu entfernen.

Ausgestaltungen des Erfindungsgegenstandes sind in den abhängigen Patentansprüchen angegeben.

Eine mit einer erfindungsgemässen Gelenkpfanne ausgestattete Hüftgelenkprothese ist Gegenstand des Patentanspruchs 8.

Die Erfindung wird anhand von in der Zeichnung schematisch dargestellten Ausführungsbeispielen erläutert. Es zeigen
- Fig. 1: Teile einer Hüftgelenkprothese mit einer erfindungsgemäss ausgebildeten Gelenkpfanne in einem diametral verlaufenden Schnitt,
- Fig. 2: eine Einzelheit der Gelenkpfanne in einer grösseren Darstellung, in einem Schnitt entsprechend der Linie II-II in Figur 1,
- Fig. 3 und 4: weitere Einzelheiten von Gelenkpfannen, je in einer der Figur 2 entsprechenden Darstellung und je in einer abgewandelten Ausführungsform.

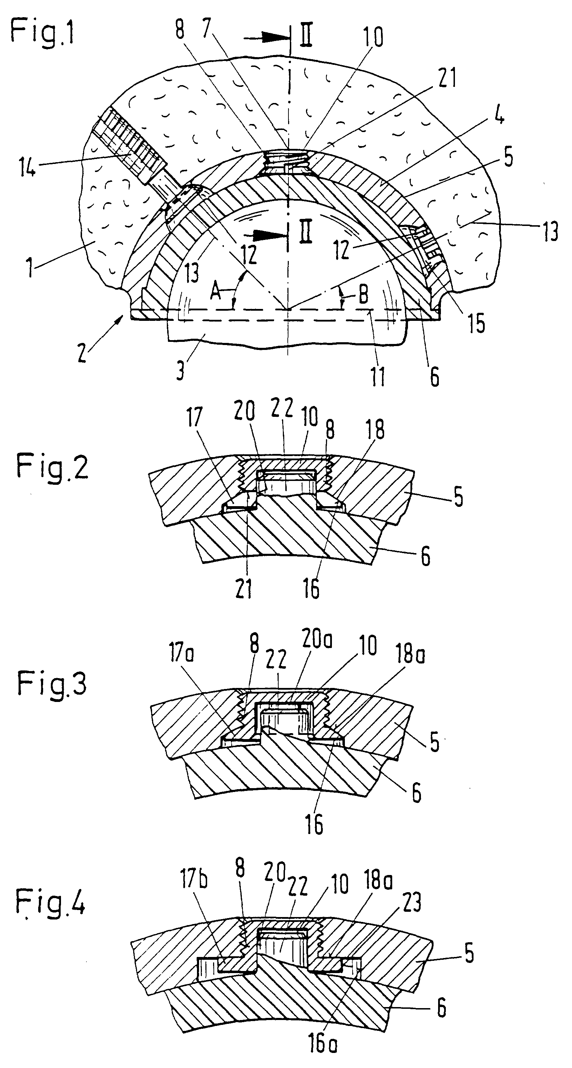

Die Hüftgelenkprothese nach den Figuren 1 und 2 enthält eine in das Knochengewebe 1 eines menschlichen Beckenteils einsetzbare Gelenkpfanne 2 und einen Gelenkkopf 3, der über einen nicht dargestellten Schaftteil in einem Oberschenkelknochen befestigbar ist. Die Gelenkpfanne 2 enthält eine in ein vorbereitetes Knochenbett 4 einführbare und in diesem verankerbare metallische, z.B. aus Titan bestehende Aussenschale 5 im wesentlichen in Form einer hohlen Halbkugel und eine in diese formschlüssig einsetzbare, ebenfalls halbkugelförmige Innenschale 6, welche den Gelenkkopf 3 aufnimmt und welche darstellungsgemäss aus einem Kunststoff, z.B. Polyäthylen, besteht. Nach einer anderen Ausführungsform kann auch eine entsprechende metallische Innenschale vorgesehen sein.

Nach einer weiteren, nicht dargestellten Ausführungsform kann zwischen der Aussenschale 5 und der Innenschale 6 auch eine Zwischenschale vorgesehen sein, die aus einem Kunststoff oder einem Metall bestehen kann.

Die Aussenschale 5 ist im Bereich ihres Pols 7 mit einer durchgehenden radialen Gewindebohrung 8 versehen, welche zur Aufnahme eines zur Positionierung der Aussenschale 5 von innen her in sie einschraubbaren Setzinstruments und eines entsprechenden Gewindestopfens 10 bestimmt ist und welche jeweils eine Kontrolle der Eindringtiefe der Aussenschale 5 gestattet. Zwischen dem Pol 7 und dem Äquator 11 der Aussenschale 5 sind weitere, in Umfangsrichtung gegeneinander versetzte radiale Bohrungen 12 vorgesehen, welche die Aussenschale 5 mit in unterschiedlichen Winkeln A und B zur Ebene des Äquators 11 geneigten Achsen 13 durchsetzen. Die Bohrungen 12 sind je zur Aufnahme eines im Knochengewebe 1 verankerbaren Befestigungselementes 14 beliebiger Art, darstellungsgemäss in Form einer in das Knochengewebe 1 einschraubbaren Knochenschraube bestimmt. Zur Befestigung der Aussenschale 5 im Knochenbett 4 können auch zwei oder mehr, z.B. drei entsprechende Befestigungselemente 14 vorgesehen sein. Die nicht benutzten, von Befestigungselementen 14 freien Bohrungen 12 können je mit einem von innen her in sie einsetzbaren und feststellbaren Verschlusselement 15 versehen sein, welches nicht Gegenstand der vorliegenden Erfindung ist.

Entsprechend der Darstellung nach den Figuren 1 und 2 ist die Gewindebohrung 8 mit einer kegeligen Ansenkung 16 versehen. Der Gewindestopfen 10 ist mit einer in diese Ansenkung 16 versenkbaren Flanschpartie 17 ausgeführt, an der eine an die Ansenkung 16 anlegbare, entsprechend kegelförmige Anpressschulter 18 ausgebildet ist. Der Gewindestopfen 10 ist ferner mit einer gegen die Innenschale 6 offenen Vertiefung 20 sowie mit Ansatzflächen für ein nicht dargestelltes Einschraubwerkzeug versehen, welche an einem die Flanschpartie 17 durchsetzenden Schlitz 21 ausgebildet sind. Die Vertiefung 20 ist gemäss Figur 2 durch eine zentrale Sackbohrung gebildet, welche zur Aufnahme eines von der Innenschale 6 abstehenden, in der Vertiefung 20 zentrierbaren zapfenartigen Vorsprungs 22 bestimmt ist. Als Einschraubwerkzeug kann ein in den Schlitz 21 einführbarer Schraubenzieher vorgesehen sein, durch den der Gewindezapfen 10 in die Gewindebohrung 8 eingeschraubt und aus dieser entfernt werden kann. Entsprechend kann einerseits auf einfache Weise eine wirksame Abdichtung der Gewindebohrung 8 erzielt und damit ein Austritt von gegebenenfalls innerhalb der Gelenkpfanne sich bildenden Abriebpartikeln in das Knochengewebe sicher verhindert werden; andererseits ist der im Bereich der Gewindebohrung 8 liegende Abschnitt des Knochenbettes 4 bei abgenommener Innenschale 6 jederzeit für eine Kontrolle der Beschaffenheit des Knochengewebes 1 zugänglich. Bei intaktem bzw. unverändertem Knochengewebe 1 kann gegebenenfalls von einer Entfernung der Aussenschale 5 abgesehen und lediglich eine neue Innenschale 6 oder eine entsprechende Zwischenschale in die Aussenschale 5 eingesetzt, durch die Gewindebohrung 8 zentriert und mit einer neuen oder gegebenenfalls der vorhandenen Gelenkkugel 3 zusammengeführt werden.

Bei der Ausführungsform nach Figur 3 ist der Gewindestopfen 10 mit einer Vertiefung 20a ausgeführt, welche einen Querschnitt in Form eines Innenpolygons, darstellungsgemäss eines Innensechskants, aufweist, wobei die Ansatzflächen für das als Steckschlüssel einführbare Einschraubwerkzeug durch die entsprechenden Flächen des Innenpolygons gebildet sind. Bei dieser Ausführung kann eine Flanschpartie 17a mit einer über den Umfang der Ansenkung 16 umlaufenden kegeligen Dichtfläche 18a vorgesehen sein, durch die eine zusätzliche, entprechend verbesserte Abbdichtung der Gewindebohrung 8 gegen die Innenseite der Aussenschale 5 erzielbar ist. Die beschriebene, in der Ansenkung 16 der Gewindebohrung 8 versenkbare Anordnung der Flanschpartie 17a erfordert eine vorteilhaft geringe Schwächung der Wandstärke der Aussenschale 5, so dass auch bei den für derartige Implantate üblichen, relativ geringen Wandstärken von z.B. 3 - 4 mm eine für eine sichere Verschraubung ausreichende Gewindelänge gewährleistet werden kann. Wie der Figur 3 weiter zu entnehmen ist, kann der Vorsprung 22 der Innenschale 6 mit einem Durchmesser ausgeführt sein, der dem Durchmesser eines dem Innenpolygon eingeschriebenen Kreises entspricht, so dass der Vorsprung 22 durch die Flächen des Innenpolygons zentrierbar ist.

Gemäss Figur 4 kann der Gewindestopfen 10 mit einer Flanschpartie 17b ausgeführt sein, welche durch Ansatzflächen 23 in Form eines Aussenpolygons, insbesondere eines Aussensechskants, begrenzt ist. Entsprechend ist der Gewindestopfen 10 über einen an diese Ansatzflächen 23 anlegbaren Schraubenschlüssel verstellbar, wobei die Gewindebohrung 8 mit einer Ansenkung 16a versehen sein kann, welche die Einführung eines auf die Flanschpartie 17b aufsteckbaren Schraubenschlüssels (Steckschlüssel) gestattet.

Zusammenfassend lässt sich die Erfindung wie folgt beschreiben:

Die Gelenkpfanne 2 enthält eine in einem Knochenbett 4 verankerbare Aussenschale 5 und eine Innenschale 6 zur Aufnahme einer Gelenkkugel 3. Die Aussenschale 5 ist in ihrem Polbereich mit einer Gewindebohrung 8 versehen, welche der Aufnahme eines zur Positionierung der Aussenschale 5 vorgesehenen Setzwerkzeugs dient und welche eine Kontrolle des darunterliegenden Teils des Knochenbettes 4 gestattet. Die Gewindebohrung 8 ist durch einen Gewindestopfen 10 gegen das Knochenbett 4 verschliessbar, wodurch ein Austritt von allfällig auftretenden, zwischen der Aussenschale 5 und der Innenschale 6 entstehenden Abriebpartikeln in das Knochengewebe 1 verhindert wird. Der Gewindestopfen 10 kann mit einer gegen die Innenschale 6 offenen Vertiefung 20 ausgeführt sein, in welcher ein von der Innenschale 6 abstehender zapfenartiger Vorsprung 22 zentrierbar ist.

## Patentansprüche

1. Mindestens zweischalige Gelenkpfanne einer Hüftgelenkprothese mit einer in einem Knochenbett (4) verankerbaren Aussenschale (5) und einer Innenschale (6) zur Aufnahme einer Gelenkkugel (3), wobei die Aussenschale (5) in ihrem Polbereich eine durchgehende Gewindebohrung (8) aufweist, welche der Aufnahme eines Setzwerkzeuges dient und die Aussenschale (5) mit einem von ihrer Innenseite in die Gewindebohrung (8) einschraubbaren Gewindestopfen (10) versehen ist, welcher die Gewindebohrung (8) gegen das Knochenbett (4) verschliesst, dadurch gekennzeichnet, dass der Gewindestopfen (10) in seinem Zentrum mit einer gegen die Innenschale (6) offenen Vertiefung (20, 20a) ausgeführt ist und die Innenschale (6) einen in diese Vertiefung (20,20a) des Gewindestopfens (10) abstehenden Vorsprung (22) aufweist, welcher eine in diese Vertiefung (20, 20a) einführbare Zentrierpartie aufweist.

2. Gelenkpfanne nach Anspruch 1, dadurch gekennzeichnet, dass der Gewindestopfen (10) Ansatzflächen für ein Einschraubwerkzeug aufweist.

3. Gelenkpfanne nach Anspruch 2, dadurch gekennzeichnet, dass die Ansatzflächen an einem den Gewindestopfen (10) durchsetzenden, gegen die Innenschale (6) offenen Schlitz (21) ausgebildet sind.

4. Gelenkpfanne nach Anspruch 2, dadurch gekennzeichnet, dass die Ansatzflächen durch in Form eines Innenpolygons, insbesondere eines Innensechskants, angeordnete Teilflächen der Vertiefung (20a) gebildet sind, und dass der Vorsprung (22) der Innenschale (6) in Form eines zwischen diesen Teilflächen zentrierbaren Zapfens ausgeführt ist.

5. Gelenkpfanne nach Anspruch 2, dadurch gekennzeichnet, dass die Ansatzflächen durch in Form eines Aussenpolygons, insbesondere eines Aussensechskants (23), angeordnete Teilflächen des Gewindestopfens (10) gebildet sind.

6. Gelenkpfanne nach einem der vorangehenden Ansprüche, dadurch gekennzeichnet, dass der Gewindestopfen (10) in einem an das Gewinde anschliessenden Endabschnitt eine Anpressschulter (18) mit einer umlaufenden, an die Aussenschale (5) anlegbaren Dichtfläche (18a) aufweist, welche die Gewindebohrung (8) zusätzlich gegen die Innenseite der Aussenschale (5) verschliesst.

7. Gelenkpfanne nach Anspruch 6, dadurch gekennzeichnet, dass die Dichtfläche (18a) des Gewindestopfens (10) an einer in eine Ansenkung (16, 16a) der Gewindebohrung (8) versenkbaren Partie der Anpresschulter (18) ausgebildet ist.

8. Hüftgelenkprothese mit einer Gelenkpfanne nach einem der vorangehenden Ansprüche.

## Claims

1. Joint socket of a hip joint prosthesis having at least two-shells and comprising an outer shell (5) anchorable in a bone seat (4) and an inner shell (6) for the receipt of a ball of a joint (3), wherein, in its pole region, the outer shell (5) comprises a throughgoing threaded bore (8) which serves for the receipt of a driver tool, and wherein the outer shell (5) is provided with a threaded plug (10) which is capable of being screwed from the inner side of the outer shell into the threaded bore (8) and which closes off the threaded bore (8) from the bone seat (4), characterised in that the threaded plug (10) is provided in its centre with a recess (20, 20a) which is open towards the inner shell (6), and in that the shell (6) has a projection (22) having a centring portion which is insertable into this recess (20, 20a).

2. Joint socket in accordance with claim 1, characterised in that the threaded plug (10) comprises contact surfaces for a screw driving tool.

3. Joint socket in accordance with claim 2, characterised in that the contact surfaces are formed on a slot (21) which passes through the threaded plug (10) and is open towards the inner shell (6).

4. Joint socket in accordance with claim 2, characterised in that the contact surfaces are formed by partial surfaces of the recess (20a) which are arranged in the form of a polygonal socket, in particular of a hexagonal socket (23), and in that the projection (22) of the inner shell (6) is executed in the form of a spigot capable of being centred between these partial surfaces.

5. Joint socket in accordance with claim 2, characterised in that the contact surfaces are formed by partial surfaces of the threaded plug (10) which are arranged in the form of a polygonal head, in particular of a hexagonal head (23).

6. Joint socket in accordance with one of the preceding claims, characterised in that the threaded plug (10) has a contact pressure shoulder (18) with a sealing surface (18a) in an end section adjacent to the thread, said contact pressure shoulder (18) being seatable onto the outer shell (5) and additionally closing off the threaded bore (8) from the inner side of the outer shell (5).

7. Joint socket in accordance with claim 6, characterised in that the sealing surface (18a) of the threaded plug (10) is formed at a portion of the contact pressure shoulder (18) which can be recessed into a counter-sink (16, 16a) of the threaded bore (8).

8. Hip joint prosthesis comprising a joint socket in accordance with one of the preceding claims.

## Revendications

1. Coque acétabulaire à au moins deux cupules d'une prothèse d'articulation de la hanche avec une cupule externe (5) pouvant être ancrée dans un lit osseux (4) et une cupule interne (6) pour la réception d'une sphère d'articulation (3), où la cupule externe (5) présente dans sa zone polaire un perçage fileté traversant (8) qui sert à la réception d'un outil de pose et où la cupule externe (5) est pourvue d'un bouchon fileté (10) pouvant être vissé depuis son côté intérieur dans le perçage fileté (8), qui ferme le perçage fileté (8) contre le lit osseux (4), caractérisée en ce que le bouchon fileté (10) est réalisé dans son centre avec un creux (20, 20a) ouvert vers la cupule interne (6) et en ce que la cupule interne (6) présente une saillie (22) dépassant dans ce creux (20, 20a) du bouchon fileté (10) qui présente une partie de centrage insérable dans ce creux (20, 20a).

2. Coque acétabulaire selon la revendication 1, caractérisée en ce que le bouchon fileté (10) présente des faces d'application pour un outil de vissage.

3. Coque acétabulaire selon la revendication 2, caractérisée en ce que les faces d'application sont réalisées à une fente (21) traversant le bouchon fileté (10), ouverte vers la cupule interne (6).

4. Coque acétabulaire selon la revendication 2, caractérisée en ce que les faces d'application sont réalisées par des faces partielles du creux (20a) agencées selon la forme d'un polygone interne, notamment d'un six pans creux, et en ce que la saillie (22) de la cupule interne (6) est réalisée sous la forme d'un ergot pouvant être centré entre ces faces partielles.

5. Coque acétabulaire selon la revendication 2, caractérisée en ce que les faces d'application sont réalisées par des faces partielles du bouchon fileté (10) agencées selon la forme d'un polygone externe, notamment d'un hexagone male (23).

6. Coque acétabulaire selon l'une des revendications précédentes, caractérisée en ce que le bouchon fileté (10) présente dans un tronçon d'extrémité faisant suite au filetage un épaulement d'application (18) avec une face d'étanchéité (18a) s'étendant tout autour, applicable à la cupule externe (5), qui ferme le perçage fileté (8) additionnellement contre le côté intérieur de la cupule externe (5).

7. Coque acétabulaire selon la revendication 6, caractérisée en ce que la face d'étanchéité (18a) du bouchon fileté (10) est réalisée à une partie de l'épaulement d'application (18) pouvant être noyée dans un chanfrein (16, 16a) du perçage fileté (8).

8. Prothèse d'articulation de la hanche avec une coque acétabulaire selon l'une des revendications précédentes.
